(19) Europäisches Patentamt
European Patent Office
Office européen des brevets

(11) Veröffentlichungsnummer: **0 113 644**
**A2**

(12) # EUROPÄISCHE PATENTANMELDUNG

(21) Anmeldenummer: 83810631.8

(22) Anmeldetag: 30.12.83

(51) Int. Cl.³: C 07 D 249/08
C 07 D 233/60, A 01 N 43/64
A 01 N 43/50
//C07D303/22, C07D303/34

(30) Priorität: 06.01.83 CH 69/83

(43) Veröffentlichungstag der Anmeldung:
18.07.84 Patentblatt 84/29

(84) Benannte Vertragsstaaten:
AT BE CH DE FR GB IT LI NL SE

(71) Anmelder: CIBA-GEIGY AG
Postfach
CH-4002 Basel(CH)

(72) Erfinder: Müller, Urs, Dr.
Schluchtstrasse 15
CH-4142 Münchenstein(CH)

(72) Erfinder: Rempfler, Hermann, Dr.
Brücklismattstrasse 16
CH-4107 Ettingen(CH)

(72) Erfinder: Tobler, Hans, Dr.
Baselmattweg 157
CH-4123 Allschwil(CH)

(54) **Halogenazolyl-propan-Derivate als mikrobizide und wuchsregulierende Mittel.**

(57) Es werden Halogenazolylpropan-Derivate der Formel I

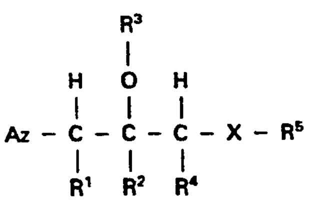

beschrieben, worin

Az Imidazolyl oder Triazolyl,

$R^1$ und $R^4$ Wasserstoff, Alkyl oder gegebenenfalls durch Halogen, Alkyl, Alkoxy, Halogenalkyl, Halogen alkoxy, Nitro, Cyan, Carboxyl oder Alkoxycarbonyl substituiertes Aralkyl, wobei nicht gleichzeitig beide Reste $R^1$ und $R^4$ für Wasserstoff stehen dürfen,

$R^2$ gegebenenfalls durch Alkoxy, Aralkoxy oder Phenyl substituiertes $C_1$-$C_{10}$-Halogenalkyl, wobei die aromatischen Kerne von Aralkoxy und Phenyl ihrerseits gegebenenfalls durch Halogen, Alkyl, Alkoxy, Halogenalkyl, Cyan, Carboxyl oder Alkoxycarbonyl substituiert sein können,

$R^3$ Wasserstoff, Alkyl, Alkylcarbonyl oder gegebenenfalls durch Halogen, Alkoxy, Alkyl, Halogenalkyl, Cyan, Carboxyl oder Alkoxycarbonyl substituiertes Aralkyl,

$R^5$ einen unsubstituierten oder ein- oder mehrfach substituierten Rest, ausgewählt aus der Reihe $C_1$-$C_8$-Alkyl, $C_3$-$C_8$-Cycloalkyl, $C_3$-$C_8$-Alkenyl, $C_3$-$C_8$-Alkinyl, Phenyl, Naphthyl, Biphenyl, Benzylphenyl, Benzyloxyphenyl, Phenoxyphenyl und Aralkyl, und

X Sauerstoff oder Schwefel bedeuten; unter Einschluss der Säureadditionssalze, quarternären Azoliumsalze und Metallkomplexe.

Es werden ferner Methoden zur Herstellung dieser Produkte offenbart sowie agrochemische Mittel, die als Wirkstoff eine dieser Verbindungen enthalten. Ferner wird ein Verfahren zur Bekämpfung phytopathogener Mikroorganismen und/oder zur Regulierung des Pflanzenwuchses mit Hilfe dieser Substanzen beschrieben.

EP 0 113 644 A2

CIBA-GEIGY AG 5-14267/-

Basel (Schweiz)

BEZEICHNUNG GEÄNDERT
siehe Titelseite

Mikrobizide und wuchsregulierende Mittel

Die vorliegende Erfindung betrifft neue, substituierte Halogenazolylpropan-Derivate sowie deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen. Weiter betrifft die Erfindung als Zwischenprodukte hergestellte Halogenazolylmethyloxirane und Halogenalkyloxirane.

Die erfindungsgemässen Halogenazolylpropan-Derivate haben die Formel I

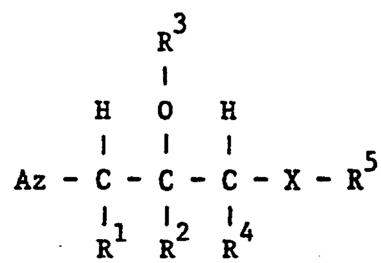

(I),

worin

Az Imidazolyl oder Triazolyl,

$R^1$ und $R^4$ Wasserstoff, Alkyl oder gegebenenfalls durch Halogen, Alkyl, Alkoxy, Halogenalkyl, Halogenalkoxy, Nitro, Cyan, Carboxyl oder Alkoxycarbonyl substituiertes Aralkyl, wobei nicht gleichzeitig beide Reste $R^1$ und $R^4$ für Wasserstoff stehen dürfen,

$R^2$ gegebenenfalls durch Alkoxy, Aralkoxy oder Phenyl substituiertes $C_1$-$C_{10}$-Halogenalkyl, wobei die aromatischen Kerne von Aralkoxy und Phenyl ihrerseits gegebenenfalls durch Halogen, Alkyl, Alkoxy, Halogenalkyl, Cyan, Carboxyl, oder Alkoxycarbonyl substituiert sein können,

$R^3$ Wasserstoff, Alkyl, Alkylcarbonyl oder gegebenenfalls durch Halogen, Alkoxy, Alkyl, Halogenalkyl, Cyan, Carboxyl oder Alkoxycarbonyl substituiertes Aralkyl,

$R^5$ einen unsubstituierten oder ein- oder mehrfach substituierten Rest, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl, Aralkyl und

X Sauerstoff oder Schwefel bedeuten; unter Einschluss der Säureadditonssalze, quaternären Azoliumsalze und Metallkomplexe.

Die vorliegende Erfindung betrifft sowohl die freien organischen Moleküle der Formel I, als auch deren Säureadditionssalze, quaternären Azoliumsalze und Metallkomplexe. Die freien Moleküle sind bevorzugt. Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Trifluormethansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz,

beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Aus der Literatur sind 1-Hydroxyäthyl-azol-Derivate als Pflanzenwachstumsregulatoren und Fungizide beispielsweise aus der europäischen Patentanmeldung 40 345 bekannt.

Der Ausdruck Azolyl kennzeichnet einen fünfgliedrigen heterocyclischen Fünfring mit Stickstoff als Heteroatom und mit aromatischem Charakter. Typische Vertreter sind 1H-1,2,4-Triazol,4H-1,2,4-Triazol und 1H-Imidazol. Unter dem Begriff Alkyl selbst oder als Bestandteil eines anderen Substituenten, wie Alkoxy, Alkylthio, Halogenalkyl, Halogenalkylthio, Aralkyl, Alkylcarbonyl, Alkylsulfonyl oder Alkylimino sind je nach Zahl der angegebenen Kohlenstoffatome beispielsweise folgende Gruppen zu verstehen: Methyl, Aethyl, Propyl, Butyl, Pentyl, Hexyl, Heptyl, Octyl, Nonyl oder Decyl, sowie ihre Isomeren, wie z.B. Isopropyl, Isobutyl, sec.-Butyl, tert.-Butyl, Isopentyl usw.. Halogenalkyl steht für einen einfach bis perhalogenierten Alkylsubstituenten, wie z.B. $CHCl_2$, $CHF_2$, $CH_2Cl$, $CCl_3$, $CH_2F$, $-CH_2CH_2Cl$, $-CHBr_2$, $-C(CH_3)_2-CH_2F$, $-C(CH_3)_2-CH_2Cl$, $-C(CH_2F)_2-CH_3$, $-C(CH_2Cl)_2-CH_3$, usw. Unter Halogen soll hier und im folgenden Fluor, Chlor, Brom oder Jod, vorzugsweise Fluor, Chlor oder Brom verstanden werden. Naphthyl steht

für α- oder β-Naphthyl, vorzugsweise α-Naphthyl. Alkenyl bedeutet z.B. Propenyl-(1), Allyl, Butenyl-(1), Butenyl-(2) oder Butenyl-(3), Alkinyl steht z.B. für Propinyl-(1) oder Propargyl. Aryl bedeutet z.B. Naphthyl, insbesondere Phenyl und Aralkyl einen Niederalkylrest, der durch eine aromatische Gruppe substituiert ist, wie z.B. Benzyl oder Phenyläthyl. Cycloalkyl bedeutet je nach Zahl der Kohlenstoffatome z.B. Cyclopropyl, Cyclobutyl, Cyclopentyl, Cyclohexyl, Cycloheptyl, Cyclooctyl usw.

Die erfindungsgemässen Verbindungen der Formel I sind bei Raumtemperatur stabile Oele, Harze oder überwiegend Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel I bevorzugt. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden und/oder mikrobiziden Wirkung sind diejenigen Verbindungen bevorzugt, in denen

Az für Imidazolyl oder 1,2,4-Triazolyl steht,

$R^1$, $R^3$ und $R^4$ unabhängig voneinander für Wasserstoff, für gegebenenfalls durch Halogen oder Cyan substituiertes $C_1$-$C_6$-Alkyl oder für gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl stehen, wobei nicht gleichzeitig die beiden Reste $R^1$ und $R^4$ für Wasserstoff stehen dürfen,

$R^2$ gegebenenfalls durch $C_1$-$C_3$-Alkoxy, Phenyl oder $C_1$-$C_3$-Phenylalkyl substituiertes $C_1$-$C_{10}$-Halogenalkyl bedeutet, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,

$R^5$ für einen unsubstituierten oder ein- oder mehrfach substituierten Rest steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, wobei die Substituenten aus der Reihe Halogen, Cyano, $C_1$-$C_3$-Alkyl, $C_1$-$C_5$-alkoxy, $C_1$-$C_5$-Haloalkyl, $C_1$-$C_3$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

Als bevorzugt können auch diejenigen Verbindungen der Formel I gelten, in denen entweder

a) Az für 1,2,4-Triazolyl steht oder
b) $R^2$ durch Fluor oder Chlor einfach oder mehrfach substituiertes $C_1$-$C_6$-Alkyl bedeuten oder
c) $R^3$ Wasserstoff oder $C_1$-$C_4$-Alkyl und einer der Reste $R^1$ oder $R^4$ $C_1$-$C_4$-Alkyl und der andere Wasserstoff oder $C_1$-$C_4$-Alkyl bedeuten oder
d) $R^5$ für durch Halogen substituiertes Phenyl steht oder
e) X Sauerstoff ist.

Weiter bevorzugt sind in der Untergruppe b) diejenigen Verbindungen, in denen $R^2$ für durch Fluor oder Chlor ein- bis zweifach substituiertes tert.-Butyl steht.

In der Untergruppe d) sind die Verbindungen weiter bevorzugt, in denen $R^5$ für in 4-Stellung durch Halogen substituiertes Phenyl steht.

Als besonders bevorzugte Untergruppe sind die Verbindungen hervorzuheben, in denen der Formel I einer der Reste $R^1$ oder $R^4$ $C_1$-$C_4$-Alkyl und der andere Wasserstoff oder $C_1$-$C_4$-Alkyl bedeuten, Az für 1,2,4-Triazolyl, $R^3$ für Wasserstoff oder $C_1$-$C_4$-Alkyl, $R^2$ für ein- bis zweifach durch Fluor oder Chlor substituiertes tert.-Butyl, $R^5$ in 4-Stellung durch Halogen substituiertes Phenyl und X für Sauerstoff stehen.

Als bevorzugte Einzelverbindungen sind zu nennen:

1-(4-Trifluormethoxyphenoxy)-2-(2-fluor-1,1-dimethyläthyl)-2-hydroxy--3-methyl-3-(1H-1,2,4-triazol-1-yl)-propan,

1-(4-Fluorphenoxy)-2-(2-fluor-1,1-dimethyläthyl)-2-hydroxy-3-methyl-3--(1H-1,2,4-triazol-1-yl)-propan und

1-(4-Chlorphenoxy)-2-(2-fluor-1,1-dimethyläthyl)-2-hydroxy-3-methyl-3--(1H-1,2,4-triazol-1-yl)-propan.

Die Verbindungen der Formel I werden nach an sich bekannten Verfahren hergestellt.

So erhält man die Verbindungen der Formel I, in denen $R^4$ Wasserstoff bedeutet, nach einem ersten Verfahren durch Reaktion eines Halogenazolylmethyloxirans der Formel II

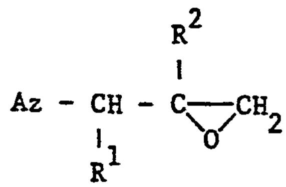 (II),

worin $R^2$ und Az die unter Formel I angegebene Bedeutung haben und $R^1$ die unter Formel I mit Ausnahme von Wasserstoff gegebene Bedeutung hat, in Gegenwart einer Base in einem inerten Lösungsmittel mit einem Alkohol oder Thioalkohol der Formel III

$$H - X - R^5 \qquad (III),$$

worin X und $R^5$ die unter Formel I gegebene Bedeutung haben, und gewünschtenfalls durch Umsetzung des Produkts der Formel Ia

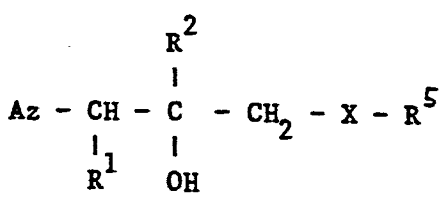 (Ia)

mit einem Verätherungs- oder Veresterungsmittel der Formel IV

$$Y - R^3 \qquad (IV),$$

worin $R^3$ die unter Formel I gegebene Bedeutung hat und Y ein Halogenatom oder einen organischen oder anorganischen Säurerest bedeutet.

Nach einem zweiten Verfahren erhält man die Verbindungen der Formel I, in denen $R^1$ Wasserstoff bedeutet, durch Reaktion eines Halogenoxirans der Formel XI

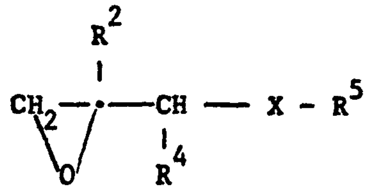

(XI),

worin X, $R^2$ und $R^5$ die unter Formel I gegebene Bedeutung haben und $R^4$ die unter Formel I mit Ausnahme von Wasserstoff gegebene Bedeutung hat, in Gegenwart einer Base in einem inerten Lösungsmittel mit einem Azol der Formel XII

$$Az - H \qquad (XII),$$

worin Az die unter Formel I gegebene Bedeutung hat, gewünschtenfalls durch Umsetzung des Produkts der Formel Ib

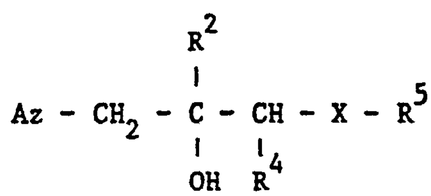

(Ib),

mit einem Verätherungs- oder Veresterungsmittel der Formel IV

$$Y - R^3 \qquad (IV),$$

worin $R^3$ die unter Formel I gegebene Bedeutung hat und Y ein Halogenatom oder einen organischen oder anorganischen Säurerest bedeutet.

Die Reaktionen (II mit III bzw. XI mit XII) werden vorteilhafterweise in Gegenwart von katalytischen Mengen an Basen als Kondensationsmittel durchgeführt. Als solche kommen organische und anorganische Basen in Betracht, z.B. tertiäre Amine wie Trialkylamine (Trimethylamin, Triäthylamin, Tripropylamin usw.), Pyridin und Pyridinbasen (4-Dimethylaminopyridin, 4-Pyrrolidylaminopyridin usw.), Oxide, Hydride und

Hydroxide, Carbonate und Hydrogencarbonate von Alkali- und Erdalkalimetallen (CaO, BaO, NaOH, LiOH, $CaH_2$, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüber hinaus eignen sich auch Alkalialkoholate wie $C_2H_5ONa$, $C_3H_7$-nONa, $(CH_3)_3CO$-K usw..

Die Reaktionen (II mit III, bzw. XI mit XII) werden bevorzugt in einem relativ polaren, jedoch reaktionsinerten organischen Lösungsmittel, durchgeführt, z.B. N,N-Dimethylformamid, N,N-Dimethylacetamid, Dimethylsulfoxid, Acetonitril, Benzonitril, Aethylenglykoldimethyläther, Diäthylenglykoldimethyläther, Triäthylenglykoldimethyläther, Dioxan, Tetrahydrofuran und anderen. Derartige Reaktionen können aber auch in Kombination mit anderen reaktionsinerten Lösungsmitteln, z.B. Benzol, Toluol, Xylol, Hexan, Petroläther, Chlorbenzol, Nitrobenzol u.a. durchgeführt werden. Die Reaktionstemperaturen liegen in einem Temperaturbereich von 20°C bis 250°C, vorzugsweise 80°C bis 180°C.

Die fakultative Umsetzung der Verbindungen der Unterformel Ia oder Ib zu denjenigen Verbindungen der Formel I, in denen $R_6$ eine andere Bedeutung als Wasserstoff hat, wird vorteilhafterweise in einem inerten organischen Lösungsmittel durchgeführt. Geeignete Lösungsmittel sind aprotische Lösungsmittel wie N,N-Dimethylformamid, N,N-Dimethylacetamid, Dimethylsulfoxid, Acetonitril, Benzonitril, N-Methylpyrrolidinon, N-Methylpiperidinon, Benzol, Toluol, Xylol, Hexan, Cyclohexan, Chlorbenzol, Nitrobenzol und andere.

Ueblicherweise steht Y für den Fall, dass die $-O-R^3$-Gruppe eine Aethergruppe ist, für Halogen wie Chlor, Brom und Jod oder für Säurereste, die sich von starken Säuren ableiten wie Schwefelsäure, Phosphorsäure, Sulfonsäuren, vorzugsweise Halogenalkylsulfonsäuren oder Halogenalkancarbonsäuren wie Trifluoressigsäure. Typische Vertreter solcher Säure-Derivate sind Dimethylsulfat, Diäthylsulfat und Trifluormethansulfonsäuremethylester. Für den Fall, dass die $-O-R^3$-Gruppe eine Estergruppe

ist, steht Y im allgemeinen für Halogen wie Chlor oder Brom oder für Säurereste von Säuren, die mit dem übertragenen Acylrest ein Anhydrid bilden können. Vorzugsweise handelt es sich dabei um Anhydride mit der gleichen Säure. Dem Reagenz Y-$R^3$ entspricht dann also z.B. Essigsäureanhydrid, Propionsäureanhydrid, Benzoesäureanhydrid, Benzolsulfonsäureanhydrid oder Trifluorsulfonsäureanhydrid.

Die Verätherung oder Veresterung der Verbindungen der Unterformel Ia geschieht mit Vorteil in Gegenwart von Basen wie Alkoholaten, Hydroxiden, Hydriden, Carbonaten oder Hydrogencarbonaten von Alkali- oder Erdalkalimetallen. Die Reaktionstemperaturen betragen 20-150°C, vorzugsweise 60-120°C.

Die Verbindungen der Formel I fallen als Diastereomerengemische an. Gegenstand der Erfindung sind alle diastereomeren Formen der Wirkstoffe der Formel I und Gemische davon. Umfasst sind somit sowohl die reinen Diastereomeren als auch die einzelnen optischen Isomeren der umfassten Enantionenpaare.

Die Alkohole oder Thioalkohole der Formel III sowie Verätherungs- und Veresterungsmittel der Formel IV sind bekannt oder werden nach an sich bekannten Methoden hergestellt.

Die Azole der Formel XII sind im Handel erhältlich. Die Oxirane der Formeln II und XI sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindung der Formel I überführen.

Die Oxirane der Formeln II und XI lassen sich durch Reaktion der zugrundeliegenden Ketone der Formel V, bzw. XIII

$$Az - \underset{\displaystyle R^1}{\underset{|}{CH}} - \overset{\displaystyle R^2}{\overset{|}{C}} = O \quad (V), \qquad O = \overset{\displaystyle R^2}{\overset{|}{C}} - \underset{\displaystyle R^4}{\underset{|}{CH}} - X - R^5 \quad (XIII),$$

worin Az, $R^2$, $R^5$ und X die unter Formel I gegebenen Bedeutungen haben und $R^1$ und $R^4$ die unter Formel I mit Ausnahme von Wasserstoff gegebene Bedeutung haben, mit Dimethylsulfoniummethylid, Dimethyloxosulfoniummethylid oder den entsprechenden Salzen wie Trimethylsulfoniumjodid oder Trimethyloxosulfoniumjodid in Gegenwart starker Basen wie Alkali- und Erdalkalialkoholate, Alkalihydroxide oder Alkali- oder Erdalkalihydride in Dimethylsulfoxid oder einem der anderen bei der Reaktion von II mit III bzw. XI mit XII beschriebenen Lösungsmittel herstellen. Unter geeigneten Umständen ist die Erzeugung des Sulfoniumylids bzw. die Reaktion des Sulfoniumsalzes mit der Base auch im Phasentransfer-Verfahren durchführbar. Als geeignete Phasentransfer-Katalysatoren wären verwendbar: quaternäre Ammoniumsalze wie Trialkyl-phenylalkylammoniumsalze oder Tetraalkylammoniumsalze, quaternäre Phosphoniumsalze wie Tetraalkylphosphoniumsalze oder Kronenäther wie 15-Krone-5 oder 18-Krone-6. Bei dieser Reaktion wird das Sulfoniumylid in situ erzeugt und reagiert direkt mit dem Keton der Formel V bzw. XIII zum Oxiran der Formel II bzw. XI. Die Reaktionen werden bei Temperaturen von 0° bis 120°C durchgeführt.

Analoge Reaktionen sind aus der Literatur bekannt; vgl. JACS, 87, 1353 (1965). Man kann die Reaktion prinzipiell analog zu den dort beschriebenen Umsetzungen vornehmen.

Die Verbindungen der Formeln V und XIII sind bekannt und zum Teil im Handel erhältlich oder können nach bekannten Methoden hergestellt werden.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder

Verdünnungsmittel anwesend sein. In Frage kommen beispielsweise aliphatische und aromatische Kohlenwasserstoffe wie Benzol, Toluol, Xylole, Petroläther; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diäthylketon, Methyläthylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon, oder in gewissen Fällen auch Kohlendioxid.

Das beschriebene Herstellungsverfahren ist, einschliesslich aller Teischritte, ein wichtiger Bestandteil der vorliegenden Erfindung.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch zu einer Förderung

des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel, wie z.B. durch Erhöhung der Photosyntheseleistung, erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall,

- zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen,also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt

werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogegen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige

Vermischen der Aktivsubstanz mit einem oder mehrereb hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer):oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein, es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblicher Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern,

Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 2 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidiertes Pflanzenöl wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit ode- zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus

natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykolähtergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propypenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash, "Encyclopedia of Surfactants", Vol. I-III, Chemical Publishing Co., New York, 1980-1981.

Die pestiziden Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25%, eines Tensides.

Insbesondere setzen sich bevorzugte Formulierungen folgendermassen zusammen: (% = Gewichtsprozent)

<u>Lösungen</u>

| | |
|---|---|
| Aktiver Wirkstoff: | 5 bis 95%, vorzugsweise 10 bis 80% |
| Lösungsmittel: | 95 bis 5%, vorzugsweise 90 bis 0% |
| oberflächenaktives Mittel: | 1 bis 30%, vorzugsweise 2 bis 20% . |

Emulgierbare Konzentrate

| | | |
|---|---|---|
| Aktiver Wirkstoff | 10 bis 50%, | bevorzugt 10 bis 40% |
| oberflächenaktives Mittel: | 5 bis 30%, | vorzugsweise 10 bis 20% |
| flüssiges Trägermittel: | 20 bis 95%, | vorzugsweise 40 bis 80%. |

Stäube

| | | |
|---|---|---|
| Aktiver Wirkstoff: | 0,5 bis 10%, | vorzugsweise 2 bis 8% |
| festes Trägermaterial | 99,5 bis 90%, | vorzugsweise 98 bis 92%. |

Suspensions-Konzentrate

| | | |
|---|---|---|
| Aktiver Wirkstoff | 5 bis 75%, | vorzugsweise 10 bis 50% |
| Wasser: | 94 bis 25%, | vorzugsweise 90 bis 30% |
| oberflächenaktives Mittel: | 1 bis 40%, | vorzugsweise 2 bis 30%. |

Benetzbare Pulver

| | | |
|---|---|---|
| Aktiver Wirkstoff: | 5 bis 90%, | vorzugsweise 10 bis 80% und insbesondere 20 bis 60% |
| oberflächenaktives Mittel: | 0,5 bis 20%, | vorzugsweise 1 bis 15% |
| festes Trägermaterial: | 5 bis 90%, | vorzugsweise 30 bis 70%. |

Granulate:

| | | |
|---|---|---|
| Aktiver Wirkstoff: | 0,5 bis 30%, | vorzugsweise 3 bis 15% |
| festes Trägermittel: | 99,5 bis 70%, | vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben.

Herstellungsbeispiele

Beispiel 1:

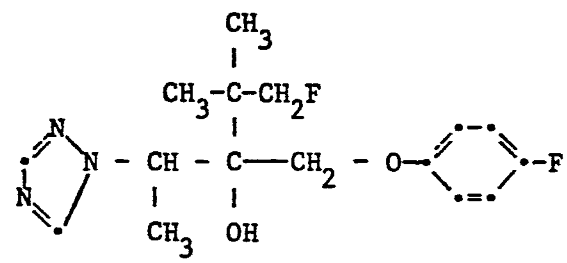

1-(4-Fluorphenoxy)-2-(2-fluor-1,1-dimethyl-äthyl)-3-methyl-3-(1H-1,2,4-triazol-1-yl)-propan (Verbindungen 6.5 und 6.22)

a) 2-(1H-1,2,4-Triazol-1-yl)-4,4-dimethyl-5-fluor-3-pentanon.

Zu einer Lösung von 5,4 g Kalium-tert.butylat in 30 ml Tetrahydrofuran lässt man eine Lösung von 8,0 g 1-(1H-1,2,4-triazol-1-yl)-3,3-dimethyl-4-fluor-3-butanon zu tropfen. Anschliessend fügt man der vorgelegten Lösung 6,7 g Methyljodid zu und rührt die Reaktionsmischung für 18 Stunden bei 50° bis 55°C. Das Reaktionsgemisch wird mit 50 ml Tetrahydrofuran verdünnt, filtriert und eingedampft. Durch Destillation des Rückstands erhält man 5,0 g 2-(1H-1,2,4-Triazol-1-yl)-4,4-dimethyl-5-fluor-3-pentanon, Sdp. 81° - 82°C / 0,013 mbar.

b) 2-[1-(1H-1,2,4-Triazol-1-yl)-äthyl]-2-(2-fluor-1,1-dimethyl-äthyl)-oxiran.

8,2 g 55%-iger Natriumhydrid-Dispersion werden mit Hexan gewaschen und anschliessend in 140 ml Dimethylsulfoxid suspendiert. Dieser Suspension setzt man portionsweise 37,75 g Trimethylsulfoxoniumjodid zu. Nachdem die Wasserstoff-Entwicklung beendet ist, lässt man eine

Lösung von 32,2 g 2-(1H-1,2,4-Triazol-1-yl)-4,4-dimethyl-5-fluor-3-pentanon in 35 ml Dimethylsulfoxid zutropfen und rührt das Gemisch für 18 Stunden bei 55° bis 60°C. Zur Aufarbeitung wird das Reaktionsgemisch mit Eiswasser aufgenommen und mit Aethylacetat extrahiert. Die organischen Phasen werden mit Wasser gewaschen, getrocknet und eingedampft. Durch Destillation des öligen Rückstands erhält man 25,4 g 2-[1-(1H-1,2,4-Triazol-1-yl)-äthyl]-2-(2-fluor-1,1-dimethyl-äthyl)-oxiran, Sdp. 92° - 94°C / 0,013 mbar.

c) Ein Gemisch von 7,5 g 2-[1-(1H-1,2,4-Triazol-1-yl)-äthyl]-2-(2-fluor-1,1-dimethyl-äthyl)-oxiran, 3,6 g 4-Fluorphenol, 0,5 g Lithiumhydroxidhydrat und 6 ml Diäthylenglykoldimethyläther wird für 7 Std. auf 145° bis 150°C erhitzt. Anschliessend wird die Mischung mit Wasser aufgenommen und mit Aethylacetat extrahiert. Die vereinigten organischen Phasen werden mit Wasser, 2N-Natriumhydroxidlösung und nochmals mit Wasser gewaschen, über Natriumsulfat getrocknet und eingedampft. Durch Chromatographie des Rückstands an Kieselgel mit Aethylacetat / Hexan-Gemisch (1:1) erhält man 2 diastereomere Fraktionen von 1-(4-Fluorphenoxy)-2-(2-fluor-1,1-dimethyl-äthyl)-3-methyl-3-(1H-1,2,4-triazol-1-yl)-propan (Verbindungen 6.5 und 6.22):

1,04 g Diastereomer I, Smp. 104° bis 105°C,
1,3 g Diastereomer II, Smp. 100° bis 102°C.

Analyse: $C_{16}H_{21}F_2N_3O_2$
Berechnet: C 59,07% H 6,51% F 11,68% N 12,92%
Gefunden : C 59,4 % H 6,4 % F 11,9 % N 13,2 %.

In analoger Weise werden die in den folgenden Tabellen aufgeführten Verbindungen hergestellt.

Tabelle 1:

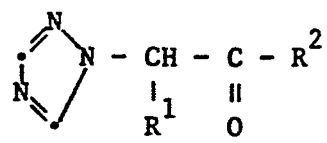

| Nr. | $R^1$ | $R^2$ | phys. Daten |
|---|---|---|---|
| 1.1 | $C_3H_7$-n | $-C(CH_3)_2-CH_2F$ | |
| 1.2 | $CH_3$ | $-C(CH_3)_2-CH_2F$ | Sdp. 87°/0,013 mb |
| 1.3 | $C_2H_5$ | $-C(CH_3)_2-CH_2F$ | |
| 1.4 | $-CO-CH_3$ | $-C(CH_3)_2-CH_2F$ | |
| 1.5 | 4-F-$C_6H_4$-$CH_2$- | $-C(CH_3(_2-CH_2F$ | |
| 1.6 | 4-Cl-$C_6H_4$-$CH_2$- | $-C(CH_3(_2-CH_2F$ | |
| 1.7 | $C_3H_7$-n | $-C(CH_2F)_2-CH_3$ | |
| 1.8 | $CH_3$ | $-C(CH_2F)_2-CH_3$ | |
| 1.9 | $C_2H_5$ | $-C(CH_2F)_2-CH_3$ | |
| 1.10 | $-CO-CH_3$ | $-C(CH_2F)_2-CH_3$ | |
| 1.11 | 4-F-$C_6H_4$-$CH_2$- | $-C(CH_2F)_2-CH_3$ | |
| 1.12 | 4-Cl-$C_6H_4$-$CH_2$- | $-C(CH_2F)_2-CH_3$ | |
| 1.13 | $C_3H_7$-n | $-C(CH_3)_2-CH_2Cl$ | |
| 1.14 | $CH_3$ | $-C(CH_3)_2-CH_2Cl$ | |
| 1.15 | $C_2H_5$ | $-C(CH_3)_2-CH_2Cl$ | |

Tabelle 2:

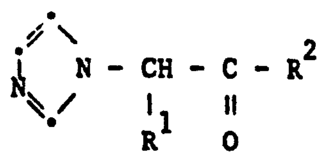

| Nr. | $R^1$ | $R^2$ | phys. Daten |
|---|---|---|---|
| 2.1 | $C_3H_7$-n | $-C(CH_3)_2-CH_2F$ | |
| 2.2 | $CH_3$ | $-C(CH_3)_2-CH_2F$ | |
| 2.3 | $C_2H_5$ | $-C(CH_3)_2-CH_2F$ | |
| 2.4 | $-CO-CH_3$ | $-C(CH_3)_2-CH_2F$ | |
| 2.5 | $4-F-C_6H_4-CH_2-$ | $-C(CH_3)_2-CH_2F$ | |
| 2.6 | $4-Cl-C_6H_4-CH_2-$ | $-C(CH_3)_2-CH_2F$ | |
| 2.7 | H | $-C(CH_2F)_2-CH_3$ | |
| 2.8 | $CH_3$ | $-C(CH_2F)_2-CH_3$ | |
| 2.9 | $C_2H_5$ | $-C(CH_2F)_2-CH_3$ | |
| 2.10 | $-CO-CH_3$ | $-C(CH_2F)_2-CH_3$ | |
| 2.11 | $4-F-C_6H_4-CH_2-$ | $-C(CH_2F)_2-CH_3$ | |
| 2.12 | $4-Cl-C_6H_4-CH_2-$ | $-C(CH_2F)_2-CH_3$ | |
| 2.13 | $C_3H_7$-n | $-C(CH_3)_2-CH_2Cl$ | |
| 2.14 | $CH_3$ | $-C(CH_3)_2-CH_2Cl$ | |
| 2.15 | $C_2H_5$ | $-C(CH_3)_2-CH_2Cl$ | |

Tabelle 3:

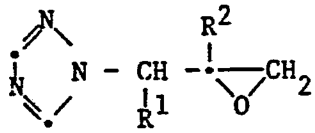

| Nr. | $R^1$ | $R^2$ | phys. Daten |
|---|---|---|---|
| 3.1 | $C_4H_9$-n | $-C(CH_3)_2-CH_2F$ | |
| 3.2 | $CH_3$ | $-C(CH_3)_2-CH_2F$ | Sdp.92-94°/0,013 mb |
| 3.3 | $C_2H_5$ | $-C(CH_3)_2-CH_2F$ | |
| 3.4 | $-CO-CH_3$ | $-C(CH_3)_2-CH_2F$ | |
| 3.5 | $4-F-C_6H_4-CH_2-$ | $-C(CH_3)_2-CH_2F$ | |
| 3.6 | $4-Cl-C_6H_4-CH_2-$ | $-C(CH_3)_2-CH_2F$ | |
| 3.7 | $C_3H_7$-n | $-C(CH_2F)_2-CH_3$ | |
| 3.8 | $CH_3$ | $-C(CH_2F)_2-CH_3$ | |
| 3.9 | $C_2H_5$ | $-C(CH_2F)_2-CH_3$ | |
| 3.10 | $-CO-CH_3$ | $-C(CH_2F)_2-CH_3$ | |
| 3.11 | $4-F-C_6H_4-CH_2-$ | $-C(CH_2F)_2-CH_3$ | |
| 3.12 | $4-Cl-C_6H_4-CH_2-$ | $-C(CH_2F)_2-CH_3$ | |
| 3.13 | $C_3H_7$-n | $-C(CH_3)_2-CH_2Cl$ | |
| 3.14 | $CH_3$ | $-C(CH_3)_2-CH_2Cl$ | |
| 3.15 | $C_2H_5$ | $-C(CH_3)_2-CH_2Cl$ | |
| 3.16 | $C_3H_7$-n | $-C(CH_3)_2-CH_2F$ | |

Tabelle 4:

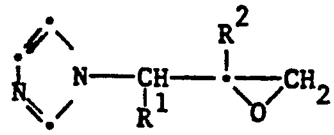

| Nr. | $R^1$ | $R^2$ | phys. Daten |
|---|---|---|---|
| 4.1 | $C_3H_7$-n | $-C(CH_3)_2-CH_2F$ | |
| 4.2 | $CH_3$ | $-C(CH_3)_2-CH_2F$ | |
| 4.3 | $C_2H_5$ | $-C(CH_3)_2-CH_2F$ | |
| 4.4 | $-CO-CH_3$ | $-C(CH_3)_2-CH_2F$ | |
| 4.5 | $4-F-C_6H_4-CH_2-$ | $-C(CH_3)_2-CH_2F$ | |
| 4.6 | $4-Cl-C_6H_4-CH_2-$ | $-C(CH_3)_2-CH_2F$ | |
| 4.7 | $C_3H_7$-n | $-C(CH_2F)_2-CH_3$ | |
| 4.8 | $CH_3$ | $-C(CH_2F)_2-CH_3$ | |
| 4.9 | $C_2H_5$ | $-C(CH_2F)_2-CH_3$ | |
| 4.10 | $-CO-CH_3$ | $-C(CH_2F)_2-CH_3$ | |
| 4.11 | $4-F-C_6H_4-CH_2-$ | $-C(CH_2F)_2-CH_3$ | |
| 4.12 | $4-Cl-C_6H_4-CH_2-$ | $-C(CH_2F)_2-CH_3$ | |
| 4.13 | $C_3H_7$-n | $-C(CH_3)_2-CH_2Cl$ | |
| 4.14 | $CH_3$ | $-C(CH_3)_2-CH_2Cl$ | |
| 4.15 | $C_2H_5$ | $-C(CH_3)_2-CH_2Cl$ | |

Tabelle 5:

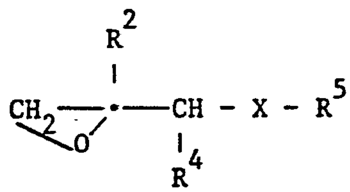

| Nr. | $R^2$ | $R^4$ | $R^5$ | X | phys. Daten |
|---|---|---|---|---|---|
| 5.1 | $-C(CH_3)_2-CH_2F$ | $CH_3$ | $4-F-C_6H_4-$ | O | Sdp. 72-74°/0,013 mb |
| 5.2 | $-C(CH_3)_2-CH_2F$ | $CH_3$ | $4-Cl-C_6H_4-$ | O | Sdp. 90-97°/0,013 mb |
| 5.3 | $-C(CH_3)_2-CH_2F$ | $CH_3$ | $4-Br-C_6H_4-$ | O | |
| 5.4 | $-C(CH_3)_2-CH_2F$ | $C_2H_5$ | $4-CH_3-C_6H_4-$ | O | |
| 5.5 | $-C(CH_3)_2-CH_2F$ | $CH_3$ | $4-CH_3-C_6H_4-$ | O | |
| 5.6 | $-C(CH_3)_2-CH_2F$ | $CH_3$ | $4-F-C_6H_4-$ | O | |
| 5.7 | $-C(CH_3)_2-CH_2F$ | $CH_3$ | $4-Cl-C_6H_4-$ | O | |
| 5.8 | $-C(CH_3)_2-CH_2F$ | $CH_3$ | $4-Br-C_6H_4-$ | O | |
| 5.9 | $-C(CH_3)-CH_2Cl$ | $CH_3$ | $4-F-C_6H_4-$ | O | |
| 5.10 | $-C(CH_3)-CH_2Cl$ | $CH_3$ | $4-Cl-C_6H_4-$ | O | |
| 5.11 | $-C(CH_3)-CH_2Cl$ | $CH_3$ | $4-Br-C_6H_4-$ | O | |
| 5.12 | $-C(CH_3)-CH_2Cl$ | $CH_3$ | $4-CH_3-C_6H_4-$ | O | |
| 5.13 | $-C(CH_2F)_2-CH_3$ | $CH_3$ | $4-F-C_6H_4-$ | O | |
| 5.14 | $-C(CH_2F)_2-CH_3$ | $CH_3$ | $4-Cl-C_6H_4-$ | O | |
| 5.15 | $-C(CH_2F)_2-CH_3$ | $CH_3$ | $4-Br-C_6H_4-$ | O | |
| 5.16 | $-C(CH_2F)_2-CH_3$ | $CH_3$ | $4-CH_3-C_6H_4-$ | O | |
| 5.17 | $-C(CH_3)_2-CH_2F$ | $CH_3$ | $4-F-C_6H_4-$ | S | |
| 5.18 | $-C(CH_3)_2-CH_2F$ | $CH_3$ | $4-Cl-C_6H_4-$ | S | |
| 5.19 | $-C(CH_3)_2-CH_2F$ | $CH_3$ | $4-CH_3-C_6H_4-$ | S | |
| 5.20 | $-C(CH_3)_2-CH_2F$ | $CH_3$ | $4-OCF_3-C_6H_4-$ | O | Sdp. 70-73°/0,013 mb |
| 5.21 | $-C(CH_3)_2-CH_2F$ | $CH_3$ | $4-CF_3-C_6H_4-$ | O | Sdp. 78-80°/0,013 mb |

Tabelle 6:

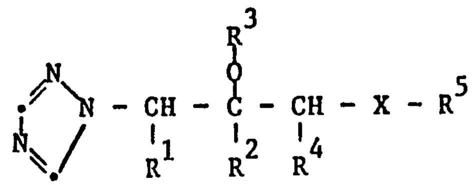

| Nr. | $R^1$ | $R^2$ | $R^3$ | $R^4$ | $R^5$ | X | phys. Daten |
|---|---|---|---|---|---|---|---|
| 6.1 | $C_3H_7$-n | $-C(CH_3)_2-CH_2F$ | H | H | 4-F-$C_6H_4$- | O | |
| 6.2 | $C_3H_7$-n | $-C(CH_3)_2-CH_2F$ | H | H | 4-Cl-$C_6H_4$- | O | |
| 6.3 | $C_2H_5$ | $-C(CH_3)_2-CH_2F$ | H | H | 4-Br-$C_6H_4$- | O | |
| 6.4 | $CH_3$ | $-C(CH_3)_2-CH_2F$ | H | H | 4-$CH_3$-$C_6H_4$- | O | |
| 6.5 | $CH_3$ | $-C(CH_3)_2-CH_2F$ | H | H | 4-F-$C_6H_4$- | O | Smp. 103° bis 104°C Isomeres I |
| 6.6 | $CH_3$ | $-C(CH_3)_2-CH_2F$ | H | H | 4-Cl-$C_6H_4$- | O | Smp. 125° bis 126°C Isomeres I |
| 6.7 | $CH_3$ | $-C(CH_3)_2-CH_2F$ | H | H | 4-Cl-$C_6H_4$- | O | Smp. 136° bis 137°C Isomeres II |
| 6.8 | $CH_3$ | $-C(CH_3)_2-CH_2F$ | H | H | 4-Br-$C_6H_4$- | O | |
| 6.9 | $C_2H_5$ | $-C(CH_3)_2-CH_2F$ | H | H | 4-F-$C_6H_4$- | O | |
| 6.10 | $-CO-CH_3$ | $-C(CH_3)_2-CH_2F$ | H | H. | 4-Cl-$C_6H_4$- | O | |
| 6.11 | 4-F-$C_6H_4$-$CH_2$- | $-C(CH_3)_2-CH_2F$ | H | H | 4-F-$C_6H_4$- | O | |
| 6.12 | 4-F-$C_6H_4$-$CH_2$- | $-C(CH_3)_2-CH_2F$ | H | H | 4-Cl-$C_6H_4$- | O | |
| 6.13 | 4-Cl-$C_6H_4$-$CH_2$- | $-C(CH_3)_2-CH_2F$ | H | H | 4-Cl-$C_6H_4$- | O | |
| 6.14 | 4-Cl-$C_6H_4$-$CH_2$- | $-C(CH_3)_2-CH_2F$ | H | H | 4-F-$C_6H_4$- | O | |
| 6.15 | $CH_3$ | $-C(CH_3)_2-CH_2F$ | H | H | 4-F-$C_6H_4$- | O | |

Tabelle 6: (Fortsetzung)

| Nr. | $R^1$ | $R^2$ | $R^3$ | $R^4$ | $R^5$ | X | phys. Daten |
|---|---|---|---|---|---|---|---|
| 6.16 | $CH_3$ | $-C(CH_2F)_2-CH_3$ | H | H | $4-Cl-C_6H_4-$ | O | |
| 6.17 | $CH_3$ | $-C(CH_2F)_2-CH_3$ | H | H | $4-F-C_6H_4-$ | O | |
| 6.18 | $CH_3$ | $-C(CH_2F)_2-CH_3$ | H | H | $4-Cl-C_6H_4-$ | O | |
| 6.19 | $CH_3$ | $-C(CH_2F)_2-CH_3$ | H | H | $4-Br-C_6H_4-$ | O | |
| 6.20 | $CH_3$ | $-C(CH_2F)_2-CH_3$ | H | H | $4-Br-C_6H_4-$ | S | |
| 6.21 | $CH_3$ | $-C(CH_3)_2-CHF_2$ | $C_6H_5-CH_2$ | H | $4-F-C_6H_4-$ | O | |
| 6.22 | $CH_3$ | $-C(CH_3)_2-CH_2F$ | H | H | $4-F-C_6H_4-$ | O | Smp.100° - 102°C Isomeres II |
| 6.23 | $CH_3$ | $-C(CH_3)_2-CH_2F$ | H | H | $4-CF_3O-C_6H_4-$ | O | Smp. 92°-93°C Isomer I |
| 6.24 | $CH_3$ | $-C(CH_3)_2-CH_2F$ | H | H | $4-CF_3O-C_6H_4-$ | O | Smp. 90° - 92°C Isomer II |
| 6.25 | H | $-C(CH_3)_2-CH_2F$ | H | $CH_3$ | $4-Cl-C_6H_4-$ | O | Smp. 89-93°C |
| 6.26 | H | $-C(CH_3)_2-CH_2F$ | H | $CH_3$ | $4-F-C_6H_4$ | O | Smp. 78-80°C |
| 6.27 | H | $-C(CH_3)_2-CH_2F$ | H | $CH_3$ | $4-CF_3-C_6H_4$ | O | Smp. 117-118°C |
| 6.28 | H | $-C(CH_3)_2-CH_2F$ | H | $CH_3$ | $4-OCF_3-C_6H_4$ | O | Smp. 104-106°C |

Tabelle 7:

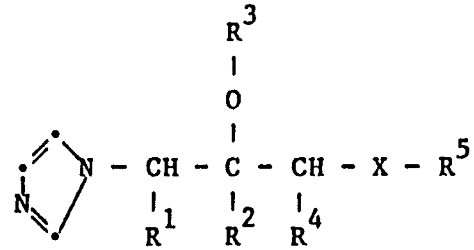

| Nr. | $R^1$ | $R^2$ | $R^3$ | $R^4$ | $R^5$ | X | phys. Daten |
|---|---|---|---|---|---|---|---|
| 7.1 | $CH_3$ | $-C(CH_3)_2-CH_2F$ | H | H | $4-F-C_6H_4-$ | O | |
| 7.2 | $CH_3$ | $-C(CH_3)_2-CH_2F$ | H | H | $4-Cl-C_6H_4-$ | O | |
| 7.3 | $CH_3$ | $-C(CH_3)_2-CH_2F$ | H | H | $4-CH_3-C_6H_4-$ | O | |
| 7.4 | $CH_3$ | $-C(CH_3)_2-CH_2F$ | H | H | $2-Cl-4-Cl-C_6H_4-$ | O | |
| 7.5 | $CH_3$ | $-C(CH_3)_2-CH_2F$ | $CH_3$ | H | $4-F-C_6H_4-$ | O | |
| 7.6 | $CH_3$ | $-C(CH_3)_2-CH_2F$ | $C_6H_5-CH_2-$ | H | $4-F-C_6H_4-$ | O | |
| 7.7 | $CH_3$ | $-C(CH_2F)_2-CH_3$ | H | H | $4-F-C_6H_4-$ | O | |
| 7.8 | $CH_3$ | $-C(CH_2F)_2-CH_3$ | H | H | $4-Cl-C_6H_4-$ | O | |

Formulierungsbeispiele

Beispiel 2:

Formulierungsbeispiele für flüssige Wirkstoffe der Formel I

(% = Gewichtsprozent)

| a) Emulsionskonzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenol-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol MG 400 | - | 70% | - | - |
| N-Methyl-2-pyrrolidon | - | 20% | - | - |
| Epoxidiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| c) Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Beispiel 3:

Formulierungsbeispiele für feste Wirkstoffe der Formel I

(% = Gewichtsprozent)

a) Spritzpulver

| | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40% |
| Aethylenglykol | 10% |
| Nonylphenolpolyäthylenglykoläther (15 Mol AeO) | 6% |

| | |
|---|---|
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel 4: Wirkung gegen Puccinia graminis auf Weizen

a) Residual-protektive Wirkung
Weizenpflanzen werden 6 Tage nach der Aussaat mit einer aus Spritzpulver des Wirkstoffes hergestellten Spritzbrühe (0,06% Aktivsubstanz) besprüht. Nach 24 Stunden werden die behandelten Pflanzen mit einer Uredosporensuspension des Pilzes infiziert. Nach einer Inkubation während 48 Stunden bei 95-100% relativer Luftfeuchtigkeit und ca. 20°C werden die infizierten Pflanzen in einem Gewächshaus bei ca. 22°C aufgestellt. Die Beurteilung der Rotpustelentwicklung erfolgt 12 Tage nach der Infektion.

b) Systemische Wirkung
Zu Weizenpflanzen wird 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100% relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus der Tabelle 1 zeigen gegen Puccinia-Pilze eine gute Wirkung. Unbehandelte aber infizierte Kontrollpflanzen zeigen einen Puccinia-Befall von 100%. Unter anderem hemmen die Verbindungen 6.2, 6.5 bis 6.7 und 6.22 den Puccinia-Befall auf 0 bis 5%.

Beispiel 5: Wirkung gegen Cercospora arachidicola auf Erdnusspflanzen

a) Residual-protektive Wirkung

10-15 cm hohe Erdnusspflanzen werden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wird eine aus Spritzpulver des Wirkstoffes hergestellte Spritzbrühe gegossen (0,06% Aktivsubstanz bezogen auf das Erdvolumen). Nach 48 Stunden werden die behandelten Pflanzen mit einer Konidiensuspension des Pilzes infiziert und 72 Stunden bei ca. 21°C und hoher Luftfeuchtigkeit inkubiert. Anschliessend werden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigen Erdnusspflanzen, die mit Wirkstoffen aus der Tabelle 1 behandelt werden, einen stark reduzierten Cercospora-Befall. So verhindern die Verbindungen 6.2, 6.5 bis 6.7 und 6.22 in obigen Versuchen das Auftreten von Flecken fast vollständig (0-10%).

Beispiel 6: Wirkung gegen Erysiphe graminis auf Gerste

a) Residual-protektive Wirkung

Ca. 8 cm hohe Gerstenpflanzen werden mit einer aus Spritzpulver des Wirkstoffes hergestellten Spritzbrühe (0,02% Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006% Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilbefall nach 10 Tagen beurteilt.

Verbindungen der Formel I zeigen eine gute Wirkung gegen Erysiphe-Pilze. Unbehandelte, aber infizierte Kontrollpflanzen zeigen einen Erysiphe-Befall von 100%. Unter anderen Verbindungen aus der Tabelle 1 hemmen die Verbindungen Nr. 6.2, 6.5 bis 6.7 und 6.22 den Pilzbefall bei Gerste auf 0 bis 5%.

Beispiel 7: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,06% Aktivsubstanz) besprüht. Nach 24 Stunden werden die behandelten Pflanzen mit einer Konidiensuspension des Pilzes infiziert. Die Pflanzen werden dann während 5 Tagen bei 90-100% relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen 6.2, 6.5 bis 6.7, 6.22 und andere hemmen den Krankheitsbefall auf weniger als 10%. Unbehandelte aber infizierte Triebe zeigen einen 100%-igen Venturia-Befall.

Beispiel 8: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02% Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgt die Beurteilung des Pilzbefalles. Die Verbindungen aus der Tabelle 1 hemmen in vielen Fällen die Pilzinfektion sehr stark. Bei einer Konzentration von 0,02% erweisen sich z.B. die Verbindungen Nr. 6.2, 6.5 und 6.22 als voll wirksam (Krankheitsbefall 0 bis 5%). Der Botrytis-Befall unbehandelter aber infizierter Bohnenpflanzen betrug 100 %.

Beispiel 9: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt umgerechnet 3,1 bzw. 0,3 kg Aktivsubstanz pro Hektar. 21 Tage nach Applikation wird das Wachstum des Getreides beurteilt. Hierbei kann festgestellt werden, dass Getreidepflanzen, die mit Wirkstoffen der Formel I behandelt worden sind, im Vergleich zu unbehandelten Kontrollpflanzen eine starke Wuchsreduktion aufweisen.

<u>Testresultate:</u>

Wuchshöhe der Getreidepflanzen in Prozent zur Wuchshöhe der unbehandelten Kontrollpflanzen.

| Verb. Nr. | 3 kg AS/ha | | 1 kg AS/ha | | 0,3 kg AS/ha | |
|---|---|---|---|---|---|---|
| | Roggen | Gerste | Roggen | Gerste | Roggen | Gerste |
| 6.6 | 17 | 30 | 37 | 62 | 68 | 89 |
| 6.7 | 19 | 40 | 39 | 79 | 69 | 100 |
| 6.22 | 5 | 11 | 17 | 30 | 31 | 57 |
| 6.23 | 76 | 89 | 86 | 96 | 86 | 96 |
| 6.25 | 51 | 56 | 76 | 82 | 98 | 95 |
| 6.26 | 43 | 46 | 72 | 77 | 94 | 85 |
| 6.27 | 78 | 74 | 104 | 92 | 92 | 95 |

Beispiel 10: Wuchshemmung bei Gräsern

In Kunststoffschalen mit Erde-Torf-Sand-Gemisch (6:3:1) wird ein Grasgemisch enthaltend Poa pratensis, Dactylis glomerata, Lolium perenne, Festuca rubra, Festuca ovina, Cynosurus crystatus, Agropyron repens und Bromus inermis im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 3,1 bzw. 0,3 kg Aktivsubstanz pro Hektar. 10 und 21 Tage nach der Applikation wird das Durchschnitts-Wachstum der Gräser beurteilt, dabei zeigt es sich, dass die erfindungsgemässen Wirkstoffe auf den Tabellen 6 und 7 eine merkliche Wuchshemmung bewirken.

Testresultate:

Wuchshöhe der Gräser in Prozent zur Wuchshöhe der unbehandelten Kontrollpflanzen.

| Verb.Nr. | 3 kg AS/ha | 1 kg AS/ha | 0,3 kg AS/ha |
|---|---|---|---|
| 6.6 | 40 | 40 | 60 |
| 6.7 | 43 | 60 | 73 |
| 6.22 | 33 | 37 | 40 |
| 6.23 | 87 | 83 | 97 |
| 6.25 | 40 | 48 | 100 |
| 6.26 | 28 | 56 | 42 |
| 6.27 | 42 | 92 | 84 |

Beispiel 11: Ertragssteigerung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifola-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt umgerechnet bis zu 3 kg Aktivsubstanz pro Hektar. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der geernteten Schoten.

Beispiel 12: Hemmung des Vegetativ-Wachstums an Soja

In Kunststofftöpfen mit einem Erde-Torf-Sandgemisch im Verhältnis 6:3:1 werden die Sojabohnen der Sorte "Hark" angesät, im Gewächshaus aufgestellt und nach Bedarf bewässert. 15 Tage nach der Saat werden die Pflanzen mit der wässrigen Spritzbrühe eines Wirkstoffes der Formel I bis zur Benetzung besprüht. Die Wirkstoffkombination beträgt umgerechnet 0,1 , 0,5 bzw. 1,5 kg Aktivsubstanz pro Hektar. 14 Tage nach der Applikation wird das Wachstum der Pflanzen beurteilt. Dabei zeigt sich, dass die erfindungsgemässen Wirkstoffe aus den Tabellen 6 und 7 eine merkliche Wuchshemmung bewirken.

Testresultate:

Wuchshöhe der Sojapflanzen in Prozent zur Wuchshöhe der unbehandelten Kontrollpflanzen.

| Verb.Nr. | 1,5 kg AS/ha | 0,5 kg AS/ha | 0,1 kg AS/ha |
|---|---|---|---|
| 6.7 | 50 | 94 | 106 |
| 6.22 | 17 | 39 | 61 |
| 6.23 | 83 | 100 | 94 |
| 6.25 | 62 | 87 | 98 |
| 6.26 | 60 | 68 | 89 |

Beispiel 13: Wuchshemmung bei Bodenbedecker-Pflanzen (Cover Crops)

In Kunststoffschalen mit Erde-Torf-Sand-Gemisch (1:1:1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70% statt. Die Testpflanzen werden auf eine Höhe von ca. 15 cm zurückgeschnitten und 5 Tage nach dem Zurückschneiden mit einer Spritzbrühe des Wirkstoffes (umgerechnet 0,1 , 0,3 , 1 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 6 und 7 eine deutliche Wuchshemmung der Bodenbedecker-Pflanzen auslösen.

Testresultate:

Neuzuwachs der Testpflanzen in Prozent Frischgewicht und in Prozent Wuchshöhe zu Neuzuwachs der unbehandelten Kontrollpflanzen. Geprüfter Wirkstoff: Verbindung Nr. 6.22

| Verb. Nr. 6.22 | kg AS/ha | Neuzuwachs | |
|---|---|---|---|
| Testpflanze | | Frischgewicht | Wuchshöhe |
| Centrosema pubescens | 3 | 0 | 0 |
| | 1 | 33 | 10 |
| | 0,3 | 30 | 15 |
| | 0,1 | 53 | 20 |
| Psophocarpus palustris | 3 | 42 | 20 |
| | 0,3 | 58 | 40 |
| | 0,1 | 65 | 50 |

Beispiel 14: Wuchsterminierung Baumwolle

In Kunststoffbehältern mit einem Erde-Torf-Gemisch im Verhältnis von 2:1 werden Baumwollpflanzen der Sorte Delta Pine angesät und im Gewächshaus bei Temperaturen von 20°-26°C angezogen. Nach 2 Monaten haben sich die Pflanzen bis zum 6 Blattstadium entwickelt. Zu diesem Zeitpunkt werden die Pflanzen mit einer wässrigen Dispersion eines Wirkstoffes der Formel I bis zur guten Benetzung besprüht. Die Wirkstoffkonzentration beträgt umgerechnet 2,0 kg Aktivsubstanz pro Hektar. Die Auswertung erfolgt ca. 1 Monat nach Applikation des Wirkstoffs. Im Vergleich zu unbehandelten Kontrollpflanzen bewirken die erfindungsgemässen Wirkstoffe der Formel I eine merkliche Reduktion des Neuzuwachses.

Patentansprüche für die Vertragsstaaten: GB, BE, DE, FR, IT, NL, SE, CH

1. Halogenazolylpropan-Derivate der Formel I

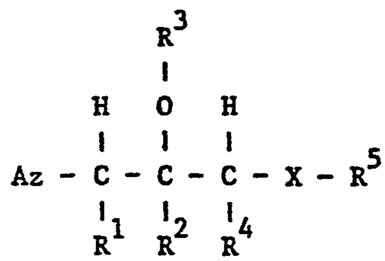

worin

Az Imidazolyl oder Triazolyl,

$R^1$ und $R^4$ Wasserstoff, Alkyl oder gegebenenfalls durch Halogen, Alkyl, Alkoxy, Halogenalkyl, Halogenalkoxy, Nitro, Cyan, Carboxyl oder Alkoxycarbonyl substituiertes Aralkyl, wobei nicht gleichzeitig beide Reste $R^1$ und $R^4$ für Wasserstoff stehen dürfen,

$R^2$ gegebenenfalls durch Alkoxy, Aralkoxy oder Phenyl substituiertes $C_1$-$C_{10}$-Halogenalkyl, wobei die aromatischen Kerne von Aralkoxy und Phenyl ihrerseits gegebenenfalls durch Halogen, Alkyl, Alkoxy, Halogenalkyl, Cyan, Carboxyl oder Alkoxycarbonyl substituiert sein können,

$R^3$ Wasserstoff, Alkyl, Alkylcarbonyl oder gegebenenfalls durch Halogen, Alkoxy, Alkyl, Halogenalkyl, Cyan, Carboxyl oder Alkoxycarbonyl substituiertes Aralkyl,

$R^5$ einen unsubstituierten oder ein- oder mehrfach substituierten Rest, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, und

X Sauerstoff oder Schwefel bedeuten; unter Einschluss der Säureadditionssalze, quarternären Azoliumsalze und Metallkomplexe.

2. Verbindungen gemäss Anspruch 1, dadurch gekennzeichnet, dass

Az für Imidazolyl oder 1,2,4-Triazolyl steht,

$R^1$, $R^3$ und $R^4$ unabhängig voneinander für Wasserstoff, für gegebenenfalls durch Halogen oder Cyan substituiertes $C_1$-$C_6$-Alkyl oder

für gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl stehen, wobei nicht gleichzeitig die beiden Reste $R^1$ und $R^4$ für Wasserstoff stehen dürfen,

$R^2$ gegebenenfalls durch $C_1$-$C_3$-Alkoxy, Phenyl oder $C_1$-$C_3$-Phenylalkyl substituiertes $C_1$-$C_{10}$-Halogenalkyl bedeutet, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,

$R^5$ für einen unsubstituierten oder ein- oder mehrfach substituierten Rest steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, wobei die Substituenten aus der Reihe Halogen, Cyano, $C_1$-$C_3$-Alkyl, $C_1$-$C_5$-alkoxy, $C_1$-$C_5$-Haloalkyl, $C_1$-$C_3$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

3. Verbindungen gemäss Anspruch 1, dadurch gekennzeichnet, dass Az für 1,2,4-Triazolyl steht.

4. Verbindungen gemäss Anspruch 1, dadurch gekennzeichnet, dass $R^2$ durch Fluor oder Chlor einfach oder mehrfach substituiertes $C_1$-$C_6$-Alkyl bedeutet.

5. Verbindungen gemäss Anspruch 4, dadurch gekennzeichnet, dass $R^2$ für durch Fluor oder Chlor ein- bis zweifach substituiertes tert.-Butyl steht.

6. Verbindungen gemäss Anspruch 1, dadurch gekennzeichnet, dass $R^3$ Wasserstoff oder $C_1$-$C_4$-Alkyl und einer der Reste $R^1$ oder $R^4$ $C_1$-$C_4$-Alkyl und der andere Wasserstoff oder $C_1$-$C_4$-Alkyl bedeuten.

7. Verbindungen gemäss Anspruch 1, dadurch gekennzeichnet, dass $R^5$ für durch Halogen substituiertes Phenyl steht.

8. Verbindungen gemäss Anspruch 1, dadurch gekennzeichnet, dass $R^5$ für in 4-Stellung durch Halogen substituiertes Phenyl steht.

9. Verbindungen gemäss Anspruch 1, dadurch gekennzeichnet, dass X Sauerstoff ist.

10. Verbindungen gemäss Anspruch 1, dadurch gekennzeichnet, dass einer der Reste $R^1$ oder $R^4$ $C_1$-$C_4$-Alkyl und der andere Wasserstoff oder $C_1$-$C_4$-Alkyl bedeuten, Az für 1,2,4-Triazolyl, $R^3$ für Wasserstoff, $R^2$ für ein- bis zweifach durch Fluor oder Chlor substituiertes tert.-Butyl, $R^5$ für in 4-Stellung durch Halogen substituiertes Phenyl und X für Sauerstoff stehen.

11. 1-(4-Trifluormethoxyphenoxy)-2-(2-fluor-1,1-dimethyläthyl)-2-hydroxy-3-methyl-3-(1H-1,2,4-triazol-1-yl)-propan gemäss Anspruch 1.

12. 1-(4-Fluorphenoxy)-2-(2-fluor-1,1-dimethyläthyl)-2-hydroxy-3-methyl-3-(1H-1,2,4-triazolyl-1-yl)-propan gemäss Anspruch 1.

13. 1-(4-Chlorphenoxy)-2-(2-fluor-1,1-dimethyläthyl)-2-hydroxy-3-methyl-3-(1H-1,2,4-triazol-1-yl)-propan gemäss Anspruch 1.

14. Halogenazolylmethyloxirane der Formel

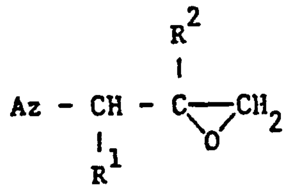

(II),

worin $R^2$ und Az die unter Formel I im Anspruch 1 gegebene Bedeutung haben und $R^1$ die unter Formel I im Anspruch 1 mit Ausnahme von Wasserstoff gegebene Bedeutung hat.

15. Halogenalkyloxirane der Formel XI

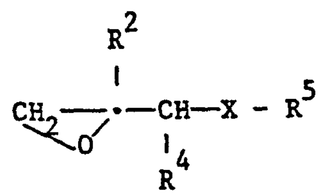

(XI),

worin X, $R^2$ und $R^5$ die unter Formel I im Anspruch 1 gegebene Bedeutung haben und $R^4$ die unter Formel I im Anspruch 1 mit ausnahme von Wasserstoff gegebene Bedeutung hat.

16. Verfahren zur Herstellung der Verbindungen der Formel I, worin $R^4$ Wasserstoff bedeutet, dadurch gekennzeichnet, dass man ein Halogenazolylmethyloxiran der Formel II

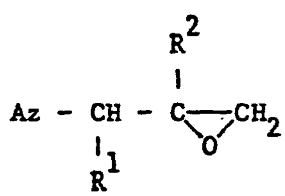

(II),

worin $R^2$ und Az die unter Formel I im Anspruch 1 angegebene Bedeutung haben und $R^1$ die unter Formel I mit Ausnahme von Wasserstoff gegebene Bedeutung hat, in Gegenwart einer Base in einem inerten Lösungsmittel mit einem Alkohol oder Thioalkohol der Formel III

$$H - X - R^5 \qquad (III),$$

worin X und $R^5$ die unter Formel I gegebene Bedeutung haben, umsetzt und das Produkt der Formel Ia

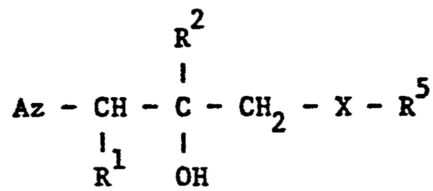 (Ia),

gewünschtenfalls durch Umsetzung mit einem Verätherungs- oder Veresterungsmittel der Formel IV

$$Y - R^3 \qquad \text{(IV)},$$

worin $R^3$ die unter Formel I gegebene Bedeutung hat und Y ein Halogenatom oder einen organischen oder anorganischen Säurerest bedeutet, veräthert oder verestert.

17. Verfahren zur Herstellung der Verbindungen der Formel I, worin $R^1$ Wasserstoff bedeutet, dadurch gekennzeichnet, dass man ein Halogenalkyloxiran der Formel XI

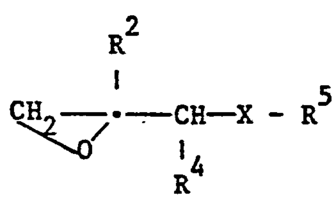 (XI),

worin X, $R^2$ und $R^5$ die unter Formel I im Anspruch 1 gegebene Bedeutung haben und $R^4$ die unter Formel I mit Ausnahme von Wasserstoff gegebene Bedeutung hat, in Gegenwart einer Base in einem inerten Lösungsmittel mit einem Azol der Formel XII

$$Az - H \qquad \text{(XII)},$$

worin Az die unter Formel I gegebene Bedeutung hat, umsetzt und das Produkt der Formel Ib

$$\begin{array}{ccccccccc} & & R^2 & & & & & & \\ & & | & & & & & & \\ Az - & CH_2 - & C & - & CH & - X - & R^5 & & (Ib), \\ & & | & & | & & & & \\ & & OH & & R^4 & & & & \end{array}$$

gewünschtenfalls durch Umsetzung mit einem Verätherungs- oder Veresterungsmittel der Formel IV

$$Y - R^3 \qquad (IV),$$

worin $R^3$ die unter Formel I gegebene Bedeutung hat und Y ein Halogenatom oder einen organischen oder anorganischen Säurerest bedeutet, veräthert oder verestert.

18. Verfahren nach Anspruch 16 oder 17, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

19. Mittel zur Bekämpfung oder Verhütung eines Befalls durch Mikroorganismen und/oder zur Regulierung des Pflanzenwachstums, dadurch gekennzeichnet, dass es neben Träger- und/oder anderen Zuschlagstoffen als Wirkstoff mindestens eine Verbindung der Formel I gemäss Anspruch 1 enthält.

20. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

21. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

22. Verfahren gemäss Anspruch 20, dadurch gekennzeichnet, dass es sich bei den Miroorganismen um phytopathogene Pilze handelt.

23. Verfahren gemäss Anspruch 20 zur Wuchshemmung im Sinne einer Erhöhung der Knickfestigkeit und Halmverkürzung bei den Getreidesorten Hafer, Weizen, Gerste oder Roggen.

24. Verfahren gemäss Anspruch 20 zur Wuchshemmung bei Gräsern und Unkräutern.

25. Verfahren gemäss Anspruch 20 zur Wuchsregulierung bei Soja im Sinne einer Ertragssteigerung.

26. Verfahren gemäss Anspruch 20 zur Wuchshemmung von Bodenbedeckerpflanzen.

27. Verfahren gemäss Anspruch 20 zur Wuchshemmung und zur Wuchsregulierung von Baumwollpflanzen im Sinne einer Ertragssteigerung.

28. Verfahren gemäss Anspruch 20 zur Wuchsregulierung bei Hafer, Weizen, Gerste oder Roggen im Sinne einer Ertragssteigerung.

29. Verfahren gemäss Anspruch 20 zur Seneszenzhemmung von Pflanzen im Sinne einer Ertragssteigerung.

FO 7.5/CW/mg*

Patentansprüche für den Vertragsstaat AT

1. Mittel zur Bekämpfung oder Verhütung eines Befalls durch Mikroorganismen und/oder zur Regulierung des Pflanzenwachstums, dadurch gekennzeichnet, dass es neben Träger- und/oder anderen Zuschlagstoffen als Wirkstoff mindestens ein Halogenazolylpropan-Derivat der Formel I

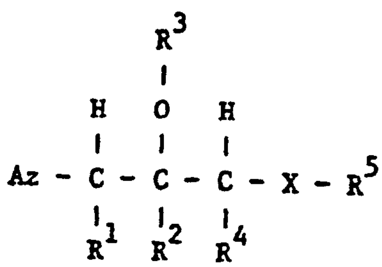

enthält, worin

Az Imidazolyl oder Triazolyl,

$R^1$ und $R^4$ Wasserstoff, Alkyl oder gegebenenfalls durch Halogen, Alkyl, Alkoxy, Halogenalkyl, Halogenalkoxy, Nitro, Cyan, Carboxyl oder Alkoxycarbonyl substituiertes Aralkyl, wobei nicht gleichzeitig beide Reste $R^1$ und $R^4$ für Wasserstoff stehen dürfen,

$R^2$ gegebenenfalls durch Alkoxy, Aralkoxy oder Phenyl substituiertes $C_1$-$C_{10}$-Halogenalkyl, wobei die aromatischen Kerne von Aralkoxy und Phenyl ihrerseits gegebenenfalls durch Halogen, Alkyl, Alkoxy, Halogenalkyl, Cyan, Carboxyl oder Alkoxycarbonyl substituiert sein können,

$R^3$ Wasserstoff, Alkyl, Alkylcarbonyl oder gegebenenfalls durch Halogen, Alkoxy, Alkyl, Halogenalkyl, Cyan, Carboxyl oder Alkoxycarbonyl substituiertes Aralkyl,

$R^5$ einen unsubstituierten oder ein- oder mehrfach substituierten Rest, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, und

X Sauerstoff oder Schwefel bedeuten; unter Einschluss der Säureadditionssalze, quarternären Azoliumsalze und Metallkomplexe.

2. Mittel gemäss Anspruch 1, dadurch gekennzeichnet, dass

Az für Imidazolyl oder 1,2,4-Triazolyl steht,

$R^1$, $R^3$ und $R^4$ unabhängig voneinander für Wasserstoff, für gegebenenfalls durch Halogen oder Cyan substituiertes $C_1$-$C_6$-Alkyl oder für gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl stehen, wobei nicht gleichzeitig die beiden Reste $R^1$ und $R^4$ für Wasserstoff stehen dürfen,

$R^2$ gegebenenfalls durch $C_1$-$C_3$-Alkoxy, Phenyl oder $C_1$-$C_3$-Phenylalkyl substituiertes $C_1$-$C_{10}$-Halogenalkyl bedeutet, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,

$R^5$ für einen unsubstituierten oder ein- oder mehrfach substituierten Rest steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, wobei die Substituenten aus der Reihe Halogen, Cyano, $C_1$-$C_3$-Alkyl, $C_1$-$C_5$-alkoxy, $C_1$-$C_5$-Haloalkyl, $C_1$-$C_3$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

3. Mittel gemäss Anspruch 1, dadurch gekennzeichnet, dass Az für 1,2,4-Triazolyl steht.

4. Mittel gemäss Anspruch 1, dadurch gekennzeichnet, dass $R^2$ durch Fluor oder Chlor einfach oder mehrfach substituiertes $C_1$-$C_6$-Alkyl bedeutet.

5. Mittel gemäss Anspruch 4, dadurch gekennzeichnet, dass $R^2$ für durch Fluor oder Chlor ein- bis zweifach substituiertes tert.-Butyl steht.

6. Mittel gemäss Anspruch 1, dadurch gekennzeichnet, dass $R^3$ Wasserstoff oder $C_1$-$C_4$-Alkyl und einer der Reste $R^1$ oder $R^4$ $C_1$-$C_4$-Alkyl und der andere Wasserstoff oder $C_1$-$C_4$-Alkyl bedeuten.

7. Mittel gemäss Anspruch 1, dadurch gekennzeichnet, dass $R^5$ für durch Halogen substituiertes Phenyl steht.

8. Mittel gemäss Anspruch 1, dadurch gekennzeichnet, dass $R^5$ für in 4-Stellung durch Halogen substituiertes Phenyl steht.

9. Mittel gemäss Anspruch 1, dadurch gekennzeichnet, dass X Sauerstoff ist.

10. Mittel gemäss Anspruch 1, dadurch gekennzeichnet, dass einer der Reste $R^1$ oder $R^4$ $C_1$-$C_4$-Alkyl und der andere Wasserstoff oder $C_1$-$C_4$-Alkyl bedeuten, Az für 1,2,4-Triazolyl, $R^3$ für Wasserstoff, $R^2$ für ein- bis zweifach durch Fluor oder Chlor substituiertes tert.-Butyl, $R^5$ für in 4-Stellung durch Halogen substituiertes Phenyl und X für Sauerstoff stehen.

11. Mittel gemäss Anspruch 1, dadurch gekennzeichnet, dass der Wirkstoff ausgewählt ist aus der Gruppe 1-(4-Trifluormethoxyphenoxy)-2-(2-fluor-1,1-dimethyläthyl)-2-hydroxy-3-methyl-3-(1H-1,2,4-triazol-1-yl)-propan,
1-(4-Fluorphenoxy)-2-(2-fluor-1,1-dimethyläthyl)-2-hydroxy-3-methyl-3-(1H-1,2,4-triazol-1-yl)-propan und
1-(4-Chlorphenoxy)-2-(2-fluor-1,1-dimethyläthyl)-2-hydroxy-3-methyl-3-(1H-1,2,4-triazolyl-1-yl)-propan.

12. Verfahren zur Herstellung der Halogenazolylmethyloxirane der Formel

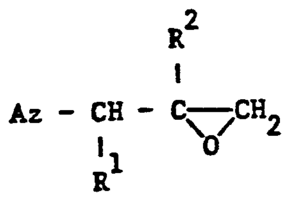 (II),

worin $R^2$ und Az die unter Formel I im Anspruch 1 gegebene Bedeutung haben und $R^1$ die unter Formel I im Anspruch 1 mit Ausnahme von Wasserstoff gegebene Bedeutung hat, dadurch gekennzeichnet, dass man ein Keton der Formel V

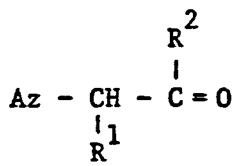 (V) ,

worin Az und $R^2$ die unter Formel I gegebenen Bedeutungen haben und $R^1$ die unter Formel I mit Ausnahme von Wasserstoff gegebene Bedeutung hat, mit Dimethylsulfoniummethylid, Dimethyloxosulfoniummethylid oder den entsprechenden Salzen in Gegenwart einer starken Base in einem polaren, reaktionsinerten Lösungsmittel umsetzt.

13. Verfahren zur Herstellung der Halogenalkyloxirane der Formel XI

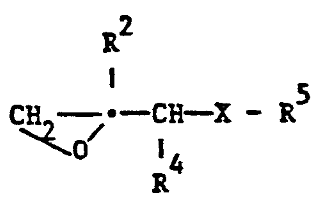 (XI),

worin X, $R^2$ und $R^5$ die unter Formel I im Anspruch 1 gegebene Bedeutung haben und $R^4$ die unter Formel I im Anspruch 1 mit ausnahme von Wasserstoff gegebene Bedeutung hat, dadurch gekennzeichnet, dass man ein Keton der Formel XIII

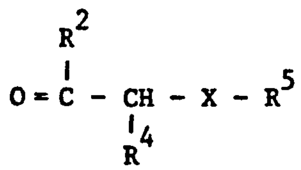 (XIII),

worin $R^2$, $R^5$ und X die unter Formel I gegebenen Bedeutungen haben und $R^4$ die unter Formel I mit Ausnahme von Wasserstoff gegebene Bedeutung hat, mit Dimethylsulfoniummethylid, Dimethyloxosulfoniummethylid oder den entsprechenden Salzen in Gegenwart einer starken Base in einem polaren, reaktionsinerten Lösungsmittel umsetzt.

14. Verfahren zur Herstellung der Verbindungen der Formel I, worin $R^4$ Wasserstoff bedeutet, dadurch gekennzeichnet, dass man ein Halogenazolylmethyloxiran der Formel II

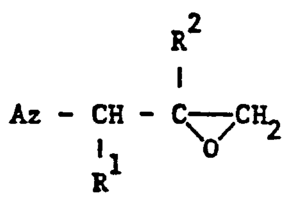 (II),

worin $R^2$ und Az die unter Formel I im Anspruch 1 angegebene Bedeutung haben und $R^1$ die unter Formel I mit Ausnahme von Wasserstoff gegebene Bedeutung hat, in Gegenwart einer Base in einem inerten Lösungsmittel mit einem Alkohol oder Thioalkohol der Formel III

$$H - X - R^5 \qquad \text{(III)},$$

worin X und $R^5$ die unter Formel I gegebene Bedeutung haben, umsetzt und das Produkt der Formel Ia

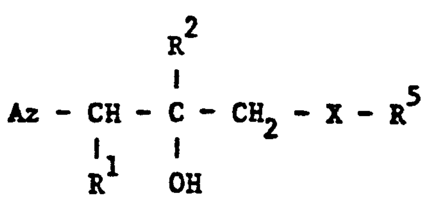 (Ia),

gewünschtenfalls durch Umsetzung mit einem Verätherungs- oder Veresterungsmittel der Formel IV

$$Y - R^3 \qquad (IV),$$

worin $R^3$ die unter Formel I gegebene Bedeutung hat und Y ein Halogenatom oder einen organischen oder anorganischen Säurerest bedeutet, veräthert oder verestert.

15. Verfahren zur Herstellung der Verbindungen der Formel I, worin $R^1$ Wasserstoff bedeutet, dadurch gekennzeichnet, dass man ein Halogenalkyloxiran der Formel XI

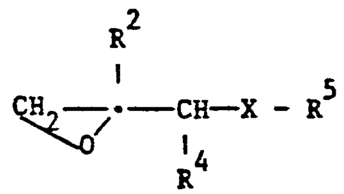

(XI),

worin X, $R^2$ und $R^5$ die unter Formel I im Anspruch 1 gegebene Bedeutung haben und $R^4$ die unter Formel I mit Ausnahme von Wasserstoff gegebene Bedeutung hat, in Gegenwart einer Base in einem inerten Lösungsmittel mit einem Azol der Formel XII

$$Az - H \qquad (XII),$$

worin Az die unter Formel I gegebene Bedeutung hat, umsetzt und das Produkt der Formel Ib

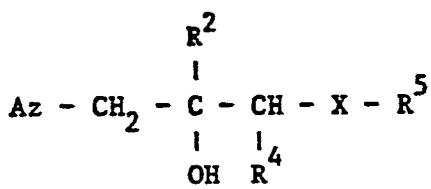

(Ib),

gewünschtenfalls durch Umsetzung mit einem Verätherungs- oder Veresterungsmittel der Formel IV

$$Y - R^3 \qquad (IV),$$

worin $R^3$ die unter Formel I gegebene Bedeutung hat und Y ein Halogenatom oder einen organischen oder anorganischen Säurerest bedeutet, veräthert oder verestert.

16. Verfahren nach Anspruch 14 oder 15, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

17. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

18. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

19. Verfahren gemäss Anspruch 17, dadurch gekennzeichnet, dass es sich bei den Miroorganismen um phytopathogene Pilze handelt.

20. Verfahren gemäss Anspruch 17 zur Wuchshemmung im Sinne einer Erhöhung der Knickfestigkeit und Halmverkürzung bei den Getreidesorten Hafer, Weizen, Gerste oder Roggen.

21. Verfahren gemäss Anspruch 17 zur Wuchshemmung bei Gräsern und Unkräutern.

22. Verfahren gemäss Anspruch 17 zur Wuchsregulierung bei Soja im Sinne einer Ertragssteigerung.

23. Verfahren gemäss Anspruch 17 zur Wuchshemmung von Bodenbedeckerpflanzen.

24. Verfahren gemäss Anspruch 17 zur Wuchshemmung und zur Wuchsregulierung von Baumwollpflanzen im Sinne einer Ertragssteigerung.

25. Verfahren gemäss Anspruch 17 zur Wuchsregulierung bei Hafer, Weizen, Gerste oder Roggen im Sinne einer Ertragssteigerung.

26. Verfahren gemäss Anspruch 17 zur Seneszenzhemmung von Pflanzen im Sinne einer Ertragssteigerung.

FO 7.5/CW/mg*